# EUROPÄISCHE PATENTSCHRIFT

(11) **EP 1 385 973 B1**
(45) Veröffentlichungstag und Bekanntmachung des Hinweises auf die Patenterteilung: **26.11.2014**
(21) Anmeldenummer: 02737997.3
(22) Anmeldetag: 25.04.2002
(51) Int. Cl.: C12P 13/02, C07C 231/06, C12M 1/02, B01J 14/00

(54) **VERFAHREN UND VORRICHTUNG ZUR HERSTELLUNG EINER WÄSSRIGEN ACRYLAMIDLÖSUNG MIT EINEM BIOKATALYSATOR**
METHOD AND DEVICE FOR PRODUCING AN AQUEOUS ACRYLAMIDE SOLUTION USING A BIOCATALYST
PROCEDE ET DISPOSITIF POUR PRODUIRE UNE SOLUTION AQUEUSE D'ACRYLAMIDE AU MOYEN D'UN BIOCATALYSEUR

(30) Priorität: 26.04.2001 DE 10120555
(43) Veröffentlichungstag der Anmeldung: 04.02.2004
(73) Patentinhaber: Ashland Licensing and Intellectual Property LLC, Dublin, OH 43017 (US)
(72) Erfinder: PETERSEN, Olaf, 60667 Meerbusch (DE); THEIS, Burkhard, 47447 Moers (DE); COLBERG, Michael, 47877 Willich (DE)
(74) Vertreter: Wolff, Felix
(86) Internationale Anmeldenummer: PCT/EP2002/004565
(87) Internationale Veröffentlichungsnummer: WO 2002/088372

(56) Entgegenhaltungen:
- EP-A- 0 150 956
- EP-A- 1 046 706
- DE-A- 2 912 292
- US-A- 5 827 699

## Beschreibung

Die vorliegende Erfindung betrifft ein Verfahren zur Herstellung einer wässrigen Acrylamidlösung durch Hydratisierung von Acrylnitril in einer wässrigen Lösung in Gegenwart eines Biokatalysators.

Die Umsetzung von Acrylnitril zu Acrylamid in Gegenwart eines geeigneten Biokatalysators in Wasser ist seit vielen Jahren bekannt und wird beispielsweise in der DE 30 17 005 C2 beschrieben, wobei bei diesem Verfahren der Biokatalysator immobilisiert ist. In der DE 44 80 132 C2 und in der EP 0 188 316 B1 werden spezielle Biokatalysatoren zur Umsetzung von Acrylnitril in Acrylamid beschrieben. Die US 5,334,519 lehrt die Hydratisierung von Acrylnitril zu Acrylamid in der Gegenwart von Biokatalysatoren und Kobalt-Ionen. Alle diese Lehren haben den Nachteil, daß ungewünschte Nebenprodukte entstehen.

DE 29 12 292 betrifft ein Verfahren zur Herstellung von Acrylamid oder Methacrylamid unter Verwendung von Mikroorganismen.

EP 0 150 956 betrifft ein Verfahren zur Herstellung von Acrylamid aus Acrylnitril unter Verwendung von Mikroorganismen.

EP 1 046 706 betrifft ein Verfahren und eine Vorrichtung zur kontinuierlichen biokatalytischen Umwandlung von wässrigen Lösungen.

Aufgabe der vorliegenden Erfindung ist es deshalb, ein möglichst umweltfreundliches Verfahren zur Verfügung zu stellen, bei dem Nebenprodukte minimiert werden.

Die Aufgabe wird erfindungsgemäß durch ein Verfahren zur Herstellung einer wässrigen Acrylamidlösung durch Hydratisierung von Acrylnitril in einer wässrigen Lösung in Gegenwart eines Biokatalysators in einer Konzentration an Biomasse bei Reaktionsbeginn von 0,03 - 2,5 g/l gelöst, wobei
(a) der Biokatalysator vor der Abtrennung von der wässrigen Acrylamidlösung geflockt wird;
(b) der Biokatalysator innerhalb von ≤ 1 Stunde nach Beendigung der Reaktion von der wässrigen Acrylamidlösung mit einer zumindest teilkontinuierlich arbeitenden, selbstentleerenden Zentrifuge abgetrennt wird, wobei der Klarlauf der Zentrifuge mit einem optischen Mittel überwacht wird und die Überwachung zur Steuerung der Zentrifuge eingesetzt wird; und
(c) der abgetrennte Biokatalysator durch zumindest einmaliges Waschen und Abtrennen von Acrylamid befreit wird, wobei das Waschwasser in den Prozess rezykliert wird.

Zum Reaktionsstart werden in dem Reaktor Wasser und der Biokatalysator vorgelegt und auf eine Temperatur von 15 bis 25 °C, vorzugsweise 16 bis 20 °C, aufgewärmt. Nachdem die Temperatur erreicht ist, wird das Acrylnitril in den Reaktor dosiert und die Umsetzung zu Acrylamid beginnt. Vorzugsweise erfolgt die gesamte Umsetzung isotherm, wobei während der gesamten Umsetzung gekühlt werden muß, um die Reaktionswärme abzuführen. Die Konzentration der Biomasse beträgt bei Reaktionsbeginn 0,03 - 2,5 g/l, bevorzugt 0,05 - 1 g/l und der pH-Wert vorzugsweise 6,0 - 8,0, besonders bevorzugt 6,8 - 7,5.

Nach Beendigung der Acrylnitrildosierung ist eine Nachreaktion von vorzugsweise 4 bis 20 Minuten, besonders bevorzugt 5 bis 10 Minuten, erforderlich, um das Acrylnitril möglichst vollständig umzusetzen.

Die Reaktion ist im Sinne der Erfindung beendet, wenn der Restgehalt an Acrylnitril in der Acrylamidlösung kleiner 10 ppm, vorzugsweise kleiner 5 ppm ist.

Erfindungsgemäß wird der Biokatalysator nach Beendigung der Reaktion innerhalb von ≤ 1 Stunde, von der wässrigen Acrylamidlösung abgetrennt.

Erfindungsgemäß erfolgt die Abtrennung des Biokatalysators mit einer zumindest teilkontinuierlich arbeitenden, selbstentleerenden Zentrifuge. Besonders bevorzugt ist diese Zentrifuge eine Ringspaltzentrifuge, wie sie beispielsweise von Dr.-Ing. Heinz Hemfort in "Separatoren", Technisch-wissenschaftliche Dokumentation, beschrieben ist.

**Erfindungsgemäß** wird der Klarlauf der Zentrifuge mit einem optischen Mittel überwacht. Dieses optische Mittel ist vorzugsweise eine Lichtschranke, die auf den gewünschten Trübungsgrad des Acrylamids im Klarlauf eingestellt ist. Die Lichtschranke ist in einer Ablaufarmatur der Zentrifuge eingebaut und durchstrahlt die ablaufende wässrige Acrylamidlösung. Die Lichtschranke besteht aus einer Lichtquelle und einem Empfänger. Die Lichtintensität der Lichtquelle wird vorzugsweise so eingestellt, daß der durch die Absorption in der durchleuchteten, wässrigen Acrylamidlösung geschwächte Lichtstrahl gerade noch am Empfänger mit einer Restintensität ankommt, die ausreicht, um dem Empfänger zu signalisieren, daß die Abtrennung des Biokatalysators hinreichend ist. Wird durch beginnende Eintrübung durch den Biokatalysator die Lichtabsorption größer, vermindert sich die Lichtintensität und der Empfänger sendet ein Signal aus, daß die Abtrennung des Biokatalysators nicht mehr ausreichend ist. Dieses Signal wird zur Steuerung der Zentrifugen eingesetzt. Vorzugsweise werden mit diesem Signal die Entleerungs- oder Reinigungsintervalle der Zentrifugen geregelt.

Erfindungsgemäß wird der Biokatalysator vor der Abtrennung geflockt. Die Flockung kann in dem Reaktor, in dem auch das Acrylnitril zu Acrylamid umgesetzt wird, erfolgen. Vorzugsweise wird die Flockung jedoch in einem separaten Flockungsbehälter durchgeführt. Die Flockung kann mit jedem geeigneten Flockungsmittel durchgeführt werden. Vorteilhafterweise wird die Flockung jedoch mit Aluminiumsulfat und/oder mit einem anionischen Polymer durchgeführt. Geeignete anionische Polymere sind beispielsweise die Produkte Praestol^{®} 2510 oder Praestol^{®} 2530 der Anmelderin.

Vorzugsweise wird die Flockung bei einem pH-Wert von 6,8 bis 8,0, besonders bevorzugt bei einem pH-Wert von 7,0 bis 7,5, durchgeführt.

Nachdem der Biokatalysator, die Biomasse, von der wässrigen Acrylamidlösung befreit worden ist, wird die Acrylamidlösung vorzugsweise auf einen pH-Wert von 4,5 bis 7,0, besonders bevorzugt von 5,5 bis 6,5, eingestellt.

### Erfindungsgemäß wird der Biokatalysator

durch zumindest einmaliges, besonders bevorzugt mehrmaliges Waschen und Abtrennen des Waschwassers von Acrylamid befreit. Vorzugsweise erfolgt das Waschen mit vollentsalztem Wasser. Ebenfalls bevorzugt wird der Biokatalysator so oft gewaschen, bis die Acrylamidkonzentration in dem Biokatalysator < 10 ppm, besonders bevorzugt < 5 ppm, beträgt.

Das mit Acrylamid beladene Waschwasser wird in den Prozeß rezykliert und beispielsweise in dem Reaktor vorgelegt. In diesem Wasser wird dann der Biokatalysator suspendiert, bevor die eigentliche Umsetzung des Acrylnitrils zu Acrylamid beginnt.

Nach dem Waschen wird der Biokatalysator vorzugsweise sterilisiert und dann als normaler Biomüll entsorgt. Die Sterilisierung erfolgt vorzugsweise durch kurzzeitiges Erhitzen des Biokatalysators auf Temperaturen > 80 °C.

Das erfindungsgemäße Verfahren kann mit jedem Biokatalysator durchgeführt werden, der die Umsetzung von Acrylnitril zu Acrylamid katalysiert. Vorzugsweise ist der Biokatalysator jedoch ein Rhodococcus rhodochrous, der unter der Hinterlegungsbezeichnung 14230 bei der DSMZ, Deutsche Sammlung von Mikroorganismen und Zellkulturen GmbH, Mascheroder Weg 1b, D-38124 Braunschweig, Deutschland, hinterlegt ist.

Das erfindungsgemäße Verfahren hat den Vorteil, daß weniger Nebenprodukte anfallen, daß die Umsetzung des Acrylnitrils zumindest nahezu vollständig erfolgt und daß eine bis zu 50 Gew.-%ige Acrylamidlösung erzielbar ist. Das erfindungsgemäße Verfahren ist einfach und kostengünstig durchzuführen. Der Biokatalysator wird optimal ausgenutzt und kann als Biomüll entsorgt werden. Das Wasser, das zum Waschen des Biokatalysators eingesetzt wird, kann in den Prozeß rezykliert werden.

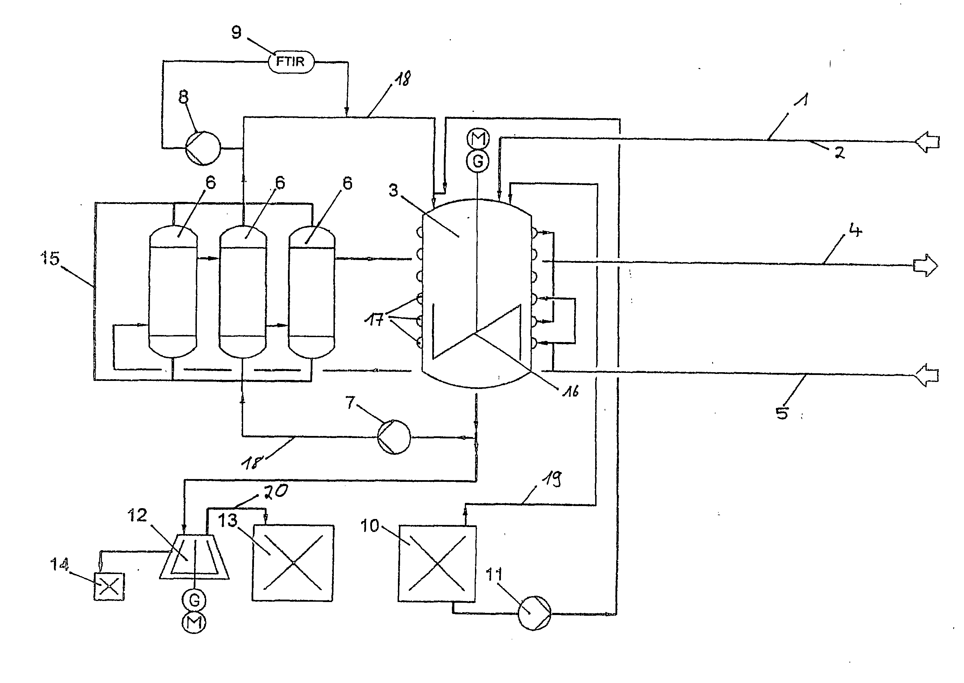

Im folgenden wird die Erfindung anhand Figur 1 erläutert. Diese Erläuterungen sind lediglich beispielhaft und schränken den allgemeinen Erfindungsgedanken nicht ein.

Figur 1 zeigt ein Verfahrensschema des erfindungsgemäßen Verfahrens bzw. Teile einer Referenzvorrichtung. Vor dem Beginn der eigentlichen Umsetzung des Acrylnitrils zu Acrylamid wird in den Reaktor 3 vollensalztes Wasser 1 und eine Suspension 2, die den Biokatalysator enthält, vorgelegt. Der Reaktor 3 wird mit einem motorgetriebenen Rührer 16 homogen durchmischt. An der Außenseite des Reaktors 3 sind Kühlschlangen 17, die mit dem Kaltwasserzulauf 5 und dem Kaltwasserrücklauf 4 verbunden sind. Der Fachmann erkennt, daß mit diesen Kühlschlangen auch das Reaktorgemisch vor dem Beginn der eigentlichen Reaktion auf eine bestimmte Temperatur vorgewärmt werden kann.

Des weiteren weist der Reaktor 3 einen Umpumpkreislauf 18 auf, durch den ein Teil des Reaktorinhalts mittels der magnetisch gekuppelten Seitenkanalpumpe 7 im Kreis gefördert wird. In dem Umpumpkreislauf 18 sind drei parallel geschaltete Rohrbündelwärmetauscher 6 angeordnet, mit denen der Reaktorinhalt erwärmt bzw. gekühlt werden kann. Die Wärmetauscher 6 sind ebenfalls mit dem Kaltwasservorlauf bzw. -rücklauf verbunden. Weiterhin weist der Umpumpkreislauf den Bypass 15 auf, mit dem die Wärmetauscher 6 umfahren werden können. Die entsprechenden Ventile sind nicht dargestellt. In dem Umpumpkreislauf ist außerdem das Fourier-Transformations-Infrarot-Gerät (FT-IR Gerät) 9 zur Online-Messung der Acrylnitril- und der Acrylamidkonzentration in dem Umlaufstrom und damit in dem Reaktor vorgesehen. Der Probenstrom wird dem Umpumpkreislauf 18 entnommen und mit der Kolben-Membranpumpe 8 in das FT-IR Gerät 9 gefördert und dort analysiert. Die Analysedaten werden zur Steuerung des Verfahrens herangezogen. Kurz bevor der Umpumpkreislauf wieder in den Reaktor eintritt, wird ihm das umzusetzende Acrylnitril mit der Membrandosierpumpe 11 aus der Acrylnitrilvorlage 10 zudosiert. Die Acrylnitrilvorlage 10 und der Reaktor 3 sind über eine Pendelleitung 19 gasseitig miteinander verbunden. Die Leitung 19 wird vor dem Dosierungsbeginn des Acrylnitrils in den Reaktor geöffnet und nach Beendigung der Dosierung wieder geschlossen. Nach Beendigung der Dosierung des Acrylnitrils ist eine Nachreaktionszeit von 5 - 20 Minuten erforderlich, um das Acrylnitril zumindest nahezu vollständig umzusetzen. Die Reaktion gilt als abgeschlossen, wenn Acrylamidkonzentration in dem Biokatalysator < 10 ppm beträgt.

Nach Abschluß der Reaktion wird Suspension in einen separaten Behälter (nicht dargestellt) gepumpt und der Biokatalysator bei einem pH-Wert von 7,0 bis 7,5 mit Aluminiumsulfat geflockt. Danach wird der Biokatalysator in einer teilkontinuierlich arbeitenden, selbstentleerenden Ringspaltzentrifuge 12 der Firma GEA Westfalia Separator AG, Werner-Habig-Straße 1, D-59302, F.R. Germany von dem Acrylamid abgetrennt, wobei die Abtrennung spätestens eine Stunde nach Beendigung der Reaktion abgeschlossen ist. Die Ringspaltzentrifuge wird mit dem Signal einer Lichtschranke (nicht dargestellt), die sich in der Leitung 20 befindet, geregelt. Insbesondere wird mit dem Signal der Lichtschranke die teilkontinuierliche Entleerung der Zentrifuge geregelt. Das wässrige Acrylamid wird in der Vorlage 13 aufgefangen und auf einen pH-Wert von 5,5 bis 6,5 eingestellt. Der Biokatalysator wird in der Vorlage 14 aufgefangen und danach mehrmals mit vollentsalztem Wasser gewaschen und entwässert, um den Biokatalysator von Acrylamid zu befreien. Das Waschwasser wird über die Leitung 1 wieder in den Prozeß rezykliert. Der gewaschene Biokatalysator wird mit Dampf sterilisiert und als Biomüll entsorgt.

## Patentansprüche

1. Verfahren zur Herstellung einer wässrigen Acrylamidlösung durch Hydratisierung von Acrylnitril in einer wässrigen Lösung in Gegenwart eines Biokatalysators in einer Konzentration an Biomasse bei Reaktionsbeginn von 0,03 - 2,5 g/l; wobei
(a) der Biokatalysator vor der Abtrennung von der wässrigen Acrylamidlösung geflockt wird;
(b) der Biokatalysator innerhalb von ≤ 1 Stunde nach Beendigung der Reaktion von der wässrigen Acrylamidlösung mit einer zumindest teilkontinuierlich arbeitenden, selbstentleerenden Zentrifuge abgetrennt wird, wobei der Klarlauf der Zentrifuge mit einem optischen Mittel überwacht wird und die Überwachung zur Steuerung der Zentrifuge eingesetzt wird; und
(c) der abgetrennte Biokatalysator durch zumindest einmaliges Waschen und Abtrennen von Acrylamid befreit wird, wobei das Waschwasser in den Prozess rezykliert wird.

2. Verfahren nach Anspruch 1, **dadurch gekennzeichnet, dass** die Zentrifuge eine Ringspaltzentrifuge ist.

3. Verfahren nach Anspruch 1 oder 2, **dadurch gekennzeichnet, dass** der Klarlauf der Zentrifuge mit einer Lichtschranke überwacht wird.

4. Verfahren nach einem der Ansprüche 1 bis 3, **dadurch gekennzeichnet, dass** in Schritt (a) als Flockungsmittel Aluminiumsulfat verwendet wird.

5. Verfahren nach einem der Ansprüche 1 bis 3, **dadurch gekennzeichnet, dass** in Schritt (a) als Flockungsmittel ein anionisches Polymer verwendet wird.

6. Verfahren nach einem der Ansprüche 1 bis 5, **dadurch gekennzeichnet, dass** die Flockung in Schritt (a) bei einem pH-Wert von 6,8 bis 8,0, vorzugsweise von 7,0 bis 7,5, durchgeführt wird.

7. Verfahren nach einem der Ansprüche 1 bis 6, **dadurch gekennzeichnet, dass** die von Biokatalysator befreite Acrylamidlösung auf einen pH-Wert von 4,5 bis 7,0, vorzugsweise 5,5 bis 6,5, eingestellt wird.

8. Verfahren nach einem der Ansprüche 1 bis 7, **dadurch gekennzeichnet, dass** das Waschen in Schritt (c) mit vollentsalztem Wasser erfolgt.

9. Verfahren nach Anspruch 8, **dadurch gekennzeichnet, dass** die Acrylamidkonzentration in dem Biokatalysator < 10 ppm, vorzugsweise < 5 ppm, beträgt.

10. Verfahren nach Anspruch 8 oder 9, **dadurch gekennzeichnet, dass** der Biokatalysator nach dem Waschen sterilisiert wird.

11. Verfahren nach einem der Ansprüche 1 bis 10, **dadurch gekennzeichnet, dass** der Biokatalysator Rhodococcus rhodochrous ist, der unter der Hinterlegungsbezeichnung 14230 bei der DSMZ, Deutsche Sammlung von Mikroorganismen und Zellkulturen GmbH, Mascheroder Weg 1b, D-38124 Braunschweig, Deutschland, hinterlegt ist.

## Claims

1. Process for producing an aqueous acrylamide solution by the hydration of acrylonitrile in an aqueous solution in the presence of a biocatalyst in a concentration of biomass at the start of the reaction from 0.03 - 2.5 g/L; whereby
(a) the biocatalyst is flocculated before the separation from the aqueous acrylamide solution;
(b) the biocatalyst is separated from the aqueous acrylamide solution within ≤ 1 hour of the end of the reaction with a self-draining, at least partially continuously operating centrifuge, whereby the clear discharge from the centrifuge is monitored using an optical means and the monitoring is used to control the centrifuge; and
(c) the separated biocatalyst is freed of acrylamide by at least a single washing and separation, whereby the washing water is recycled in the process.

2. The process according to claim 1, **characterized in that** the centrifuge is an annular gap centrifuge.

3. The process according to claim 1 or 2, **characterized in that** the clear discharge from the centrifuge is monitored with a light barrier.

4. The process according to anyone of claims 1 to 3, **characterized in that** in step (a) aluminum sulphate is used as the flocculation agent.

5. The process according to anyone of claims 1 to 3, **characterized in that** in step (a) an ionic polymer is used as the flocculation agent.

6. The process according to anyone of claims 1 to 5, **characterized in that** the flocculation in step (a) is performed at the pH value of 6.8 to 8.0, preferably 7.0 to 7.5.

7. The process according to anyone of claims 1 to 6, **characterized in that** the aqueous acrylamide solution freed of biocatalyst is set to a pH value of 4.5 to 7.0, preferably 5.5 to 6.5.

8. The process according to anyone of claims 1 to 7, **characterized in that** the washing in step (c) is performed with deionized water.

9. The process according to claim 8, **characterized in that** the acrylamide concentration in the biocatalyst is < 10 ppm, preferably < 5 ppm.

10. The process according to claim 8 or 9, **characterized in that** the biocatalyst is sterilized after the washing.

11. The process according to anyone of claims 1 to 10, **characterized in that** the biocatalyst is Rhodococcus rhodochrous, filed under the deposition number 14230 with DSMZ, Deutsche Sammlung von Mikroorganismen und Zellkulturen GmbH, Mascheroder Weg 1 b, D-38124 Braunschweig, Germany.

## Revendications

1. Procédé pour la production d'une solution aqueuse d'acrylamide par hydratation d'acrylonitrile dans une solution aqueuse en présence d'un biocatalyseur à une concentration de biomasse au début de la réaction de 0,03 - 2,5 g/l ; dans lequel
(a) on fait floculer le biocatalyseur avant la séparation d'avec la solution aqueuse d'acrylamide ;
(b) on sépare le biocatalyseur, en l'espace de ≤ 1 heure après la fin de la réaction, de la solution aqueuse d'acrylamide, à l'aide d'une centrifugeuse à auto-évacuation fonctionnant au moins partiellement en continu, le flux clarifié de la centrifugeuse étant contrôlé à l'aide d'un moyen optique et le contrôle étant utilisé pour la commande de la centrifugeuse ; et
(c) on libère le biocatalyseur séparé, par au moins un lavage et séparation d'acrylamide, l'eau de lavage étant recyclée dans le processus.

2. Procédé selon la revendication 1, **caractérisé en ce que** la centrifugeuse est une centrifugeuse à espace annulaire.

3. Procédé selon la revendication 1 ou 2, **caractérisé en ce que** le flux clarifié de la centrifugeuse est contrôlé à l'aide d'une barrière photoélectrique.

4. Procédé selon l'une quelconque des revendications 1 à 3, **caractérisé en ce que** dans l'étape (a) on utilise comme floculant du sulfate d'aluminium.

5. Procédé selon l'une quelconque des revendications 1 à 3, **caractérisé en ce que** dans l'étape (a) on utilise comme floculant un polymère anionique.

6. Procédé selon l'une quelconque des revendications 1 à 5, **caractérisé en ce qu'**on effectue la floculation dans l'étape (a) à un pH de 6, 8 à 8,0, de préférence de 7,0 à 7,5.

7. Procédé selon l'une quelconque des revendications 1 à 6, **caractérisé en ce qu'**on ajuste à un pH de 4,5 à 7,0, de préférence de 5,5 à 6,5, la solution d'acrylamide libérée de biocatalyseur.

8. Procédé selon l'une quelconque des revendications 1 à 7, **caractérisé en ce que** le lavage dans l'étape (c) s'effectue à l'aide d'eau totalement déminéralisée.

9. Procédé selon la revendication 8, **caractérisé en ce que** la concentration d'acrylamide dans le biocatalyseur est < 10 ppm, de préférence < 5 ppm.

10. Procédé selon la revendication 8 ou 9, **caractérisé en ce qu'**après le lavage on stérilise le biocatalyseur.

11. Procédé selon l'une quelconque des revendications 1 à 10, **caractérisé en ce que** le biocatalyseur est *Rhodococcus rhodochrous,* qui est déposé sous le numéro de dépôt 14230 à la DSMZ, Deutsche Sammlung von Mikroorganismen und Zellkulturen GmbH, Mascheroder Weg 1b, D-38124 Braunschweig, Allemagne.
